# EUROPÄISCHE PATENTSCHRIFT

(11) **EP 1 280 788 B1**
(45) Veröffentlichungstag und Bekanntmachung des Hinweises auf die Patenterteilung: **22.09.2004**
(21) Anmeldenummer: 01931684.3
(22) Anmeldetag: 03.05.2001
(51) Int. Cl.: C07D 307/62

(54) **VERFAHREN ZUR HERSTELLUNG VON L-ASCORBINSÄURE DURCH LACTONISIERUNG VON 2-KETO-L-GULONSÄURE ODER 2-KETO-L-GULONSÄUREESTERN**
METHOD FOR THE PRODUCTION OF L-ASCORBIC ACID BY LACTONISATION OF 2-KETO-L-GULONIC ACID OR 2-KETO-L-GULONATE ESTERS
PROCEDE DE PREPARATION D'ACIDE L-ASCORBIQUE PAR LACTONISATION D'ACIDE 2-CETO-L-GULONIQUE OU D'ESTERS D'ACIDE 2-CETO-L-GULONIQUE

(30) Priorität: 10.05.2000 DE 10022518
(43) Veröffentlichungstag der Anmeldung: 05.02.2003
(73) Patentinhaber: BASF AKTIENGESELLSCHAFT, 67056 Ludwigshafen (DE)
(72) Erfinder: BÖTTCHER, Andreas, 69226 Nussloch (DE); BURST, Wolfram, 68199 Mannheim (DE)
(86) Internationale Anmeldenummer: PCT/EP2001/004961
(87) Internationale Veröffentlichungsnummer: WO 2001/085711

(56) Entgegenhaltungen:
- WO-A-99/03853
- WO-A-99/07691
- DE-A- 2 939 052
- US-A- 2 185 383
- US-A- 2 462 251

## Beschreibung

Die Erfindung betrifft ein Verfahren zur Herstellung von L-Ascorbinsäure, bei dem freie 2-Keto-L-gulonsäure oder 2-Keto-L-gulonsäure-C₃-C₁₀-alkylester unter sauren Bedingungen in Gegenwart eines mit Wasser mischbaren Lösungsmittels lactonisiert wird und wobei dieses Lösungsmittel in situ ein Lösungsmittel bildet, in dem die gebildete Ascorbinsäure schwer löslich ist.

Zur Herstellung von L-Ascorbinsäure sind in der Vergangenheit eine Vielzahl von Verfahrensvarianten veröffentlicht worden. Eine Übersicht findet sich u.a. in Crawford et al., Adv. Carbohydrate Chem. 37, 79 (1980) sowie in Ullmann's Encyclopedia of Industrial Chemistry, Vol. A27, 551-557 (1996).

Bekannt sind eine Reihe von Verfahren zur Herstellung von Ascorbinsäure durch Umsetzung von 2-Keto-L-gulonsäure mit einer Säure.

So ist in der US 2,185,383 die Umsetzung von 2-Keto-L-gulonsäure mit konzentrierter Salzsäure und Essigsäure als Lösungsmittel beschrieben.

JP-OS 58-177986 beschreibt ein Verfahren, das die Zugabe von Ethanol und Aceton zu dem Natrium-Salz der 2-Keto-L-gulonsäure, die Neutralisation mit Salzsäure, die Abtrennung des ausgefällten Natriumchlorids durch Filtration und anschließend das Halten der Reaktionsmischung bei Temperaturen im Bereich von 25°C bis 75°C umfaßt, wodurch L-Ascorbinsäure erhalten wird.

In der JP-AS 48-15931 wird die Umsetzung von 2-Keto-L-gulonsäure mit einer Mineralsäure in einem inerten Lösungsmittel in Gegenwart einer oberflächenaktiven Substanz beschrieben.

WO 87/00839 beansprucht ein Verfahren, bei dem eine Aufschlämmung von 2-Keto-L-gulonsäure in einem inerten organischen Lösungsmittel in Gegenwart eines grenzflächenaktiven Mittel unter Säurekatalyse zu L-Ascorbinsäure umgesetzt wird.

DE-A-195 47 073 beschreibt ein Verfahren zur Herstellung von L-Ascorbinsäure durch Umsetzung von 2-Keto-L-gulonsäure mit wäßriger Mineralsäure in einem Lösungsmittelgemisch, enthaltend ein inertes organisches Lösungsmittel, ein aliphatisches Keton sowie ein Säurechlorid.

WO 99/07691 beschreibt die Umsetzung von 2-Keto-L-gulonsäure mit konzentrierter Salzsäure bei Temperaturen zwischen 40 und 80°C.

EP-A-0 671 405 offenbart ein Verfahren zur Herstellung von 2-Keto-L-gulonsäure-methyl- oder -ethylester durch Veresterung von 2-Keto-L-gulonsäure mit Methanol bzw. Ethanol in Gegenwart eines sauren Ionenaustauschers. Ferner ist in dieser Anmeldeschrift zu lesen, daß die o.g. Ester einer alkalischen Umlagerung (Lactonisierung) zur Ascorbinsäure oder zu einem Salz davon unterworfen werden können.

US 5,391,770 beschreibt die Veresterung von 2-Keto-L-gulonsäure mit anschließender basenkatalysierter Lactonisierung der gebildeten Ester zu Salzen der L-Ascorbinsäure und Freisetzung der Ascorbinsäure durch Zugabe einer starken Säure.

In der japanischen Auslegeschrift 22 113/75 wird die Veresterung von 2-Keto-L-gulonsäure mit Butanol und die anschließende säurekatalysierte Lactonisierung in Benzol als Lösungsmittel beschrieben.

Die oben genannten Ausführungsformen der sauer katalysierte Umlagerung von 2-Keto-L-gulonsäure ist aufgrund ihrer langen Reaktionszeit und möglichen Folgereaktionen der gebildeten Ascorbinsäure wirtschaftlich weniger attraktiv. So ist in der Regel die Verwendung eines inerten Lösungsmittels unumgänglich, um die Folgereaktionen der Ascorbinsäure mit wäßriger Salzsäure zu unterdrücken. Insbesondere die vollständige Abtrennung des Katalysators Chlorwasserstoff erfordert einen hohen technischen Aufwand, der meist mit der Verwendung eines speziellen Lösungsmittels einhergeht. Dennoch wird diese Verfahrensweise technisch sehr breit eingesetzt. Bei der sauer katalysieren Lactonisierung von 2-Keto-L-gulonsäure, ihrem Ester oder ihrer Isopropyliden-geschützten Form ist man aus bekannten Gründen auf den Einsatz bestimmter Lösungsmittel angewiesen. Als geeignete Lösungsmittel werden für diese diskontinuierliche Umlagerung in Lösung vorteilhafter Weise unpolare halogenierte oder nichthalogenierte Kohlenwasserstoffe, wie z.B. Tetrachlorkohlenstoff, Chloroform, Dichlorethan, 1,2-Trichlorethylen, Perchlorethylen oder aromatische Kohlenwasserstoffe, wie Toluol, Chlorbenzol, Benzol oder Xylol eingesetzt. Mit gutem Erfolg können auch cyclische Carbonate, wie z.B. Propylencarbonat, verwendet werden. Diese inerten Lösungsmittel führen in der anschließenden Reinigung des Reaktionsproduktes Ascorbinsäure zu Aufarbeitungsproblemen, da sie häufig destillativ nur mit hohem Aufwand trennbare Azeotrope bilden und sich durch verdampfung oder Strippung nur schwer vom Wertprodukt vollständig abtrennen lassen. Diese Destillationen sind mit Produktverlust und einer Belastung der Umwelt verbunden. Nicht rückgewonnene Lösungsmittel müssen außerdem in reiner Form dem Prozeß wieder zugeführt werden.

Eine weitere Schwierigkeit ist, daß die 2-Keto-L-gulonsäure zu Beginn und im Verlauf der Reaktion stets ungelöst in Form einer Suspension vorliegt und eine Reaktion nur an der Kristalloberfläche erfolgt. Der Zusatz von grenzflächenaktiven Substanzen ändert am Reaktionsverlauf nur wenig. Vielmehr kann dieser Hilfsstoff nur mühsam vom Rohprodukt abgetrennt werden und bedeutet zusätzliche Aufreinigungsschritte, um die gewünschte Reinheit der L-Ascorbinsäure zu erzielen. Nachteilig sind weiterhin lange Reaktionszeiten und demzufolge große Apparatevolumina.

Die bei der sauer katalysierten Umlagerung von 2-Keto-L-gulonsäure auftretenden Nebenreaktionen der Ascorbinsäure mit dem sauren Katalysator sind hinlänglich bekannt. Es gibt deshalb kein technisches Verfahren, das ohne den Einsatz einer technisch aufwendigen Vorreinigung, z.B. durch eine Aktivkohlereinigung vor der Hochreinigung der Ascorbinsäure, auskommt. Je nach Verfahrensvariante ist die Standzeit dieses Kohlefilters unterschiedlich. Meist senkt sie aber die Wirtschaftlichkeit des Gesamtverfahrens.

Kontinuierliche Lactonisierungen von 2-Keto-L-gulonsäure und Diaceton-2-keto-L-gulonsäure werden in US 2,462,251; DE 29 39052; GB 601789 und GB 1222322 beschrieben. Von Nachteil ist bei diesen Verfahren, daß die Katalysatorrückgewinnung, insbesondere die Aufkonzentrierung der wäßrigen Katalysatorlösung, aufwendig und damit wirtschaftlich unvorteilhaft ist.

In DE 2939052 wird die kontinuierliche Lactonisierung von 2-Keto-L-gulonsäure in Gegenwart von wäßriger Salzsäure bei Temperaturen über 80°C und Zusatz von niederen Alkoholen nach der Enolisierung beschrieben. Durch Quenchen des heißen Reaktionsgemisches mit Butanol wird eine rasche Abkühlung und weitgehende Abtrennung des Katalysators als Azeotrop bewirkt. Die Rückführung der Salzsäure ist nur mit einem aufwendigen und teuerem Verfahrenskonzept, wie oben beschrieben, zu realisieren.

Es bestand daher die Aufgabe, ein Verfahren zur Herstellung von L-Ascorbinsäure bereitzustellen, welches die oben genannten Nachteile nicht aufweist.

Diese Aufgabe wurde durch ein Verfahren zur Herstellung von L-Ascorbinsäure, dadurch gekennzeichnet, daß man 2-Keto-L-gulonsäure oder eine Schmelze von 2-Keto-L-gulonsäure-C₃-C₆-alkylester unter Säurekatalyse in Gegenwart ausschließlich eines Lösungsmittels oder Lösungsmittelgemisches lactonisiert, das mit Wasser mischbar ist und in situ ein Lösungsmittel bildet, in dem die gebildete Ascorbinsäure schwer löslich ist.

Ferner ist das erfindungsgemäße Verfahren in einer bevorzugten Ausführungsform dadurch gekennzeichnet, daß es folgende Schritte umfaßt:
a) Veresterung von 2-Keto-L-gulonsäure oder 2,3:4,6-Di-O-isopropyliden-2-keto-L-gulonsäure in Gegenwart eines sauren Katalysators mit einem C₃-C₆-Alkohol,
b) Destillation des überschüssigen C₃-C₆-Alkohols zusammen mit dem gebildeten Reaktionswasser und
c) Lactonisierung des gebildeten 2-Keto-L-gulonsäure-C₃-C₆-alkylester unter Säurekatalyse in Gegenwart ausschließlich eines Lösungsmittels oder Lösungsmittelgemisches, das mit Wasser mischbar ist und in situ ein Lösungsmittel bildet, in dem die gebildete Ascorbinsäure schwer löslich ist.

Im Rahmen des erfindungsgemäßen Verfahrens wird zunächst 2-Keto-L-gulonsäure oder 2,3:4,6-Di-O-isopropyliden-2-keto-L-gulonsäure in einem einstufigen Veresterungsschritt in Gegenwart eines sauren Katalysators zum Alkylester umgesetzt. Die Veresterung erfolgt in einem Temperaturbereich von -10 bis 160°C, vorzugsweise von 20 bis 100°C, besonders bevorzugt in einem Temperaturbereich von 40 bis 95°C.

Zur Veresterung eignen sich vorteilhafterweise höhere Alkylester aus gesättigten, verzweigten oder unverzweigten Alkylalkoholen mit einer Kohlenstoffanzahl größer gleich 3, bevorzugt mit einem Alkylrest von 3 bis 10 Kohlenstoffatomen, wie z.B. n-Propanol, Isopropanol, 1-Butanol, 2-Butanol, 2-Methyl-1-propanol, 2-Methyl-2-propanol, 1-Pentanol, 2-Pentanol, 3-Pentanol, 1-Hexanol, 2-Hexanol, 1-Heptanol, 2-Heptanol, 1-Octanol, 2-Octanol, 3-Octanol, 1-Nonanol, 2-Nonanol, 1-Decanol, 2-Decanol, 4-Decanol.

Vorzugsweise werden solche Alkohole für die Veresterung eingesetzt, in denen L-Ascorbinsäure schwer löslich ist. Besonders bevorzugt sind C₄-C₆-Alkohole, ausgewählt aus der Gruppe, bestehend aus n-Propanol, Isopropanol, 1-Butanol, 2-Methyl-1-propanol, 2-Methyl-2-propanol, 1-Pentanol oder 1-Hexanol, ganz besonders sind 1-Butanol und 1-Pentanol geeignet.

Der Alkohol wird dabei in einem 2- bis 10-fachen, bevorzugt 3- bis 6-fachen molaren Überschuß, bezogen auf die eingesetzte 2-Keto-L-gulonsäure bzw. 2,3:4,6-Di-O-isopropyliden-2-keto-Lgulonsäure, eingesetzt.

Für die Synthese wird bevorzugt 2-Keto-L-gulonsäure als Ausgangsmaterial eingesetzt. Die Säure kann dabei sowohl in kristalliner Form, beispielsweise als getrocknetes oder schleuderfeuchtes Monohydrat oder als wasserfreie Verbindung als auch als wäßrige Lösung, beispielsweise als aufkonzentrierte Fermentationslösung eingesetzt werden.

Das Monohydrat der 2-Keto-L-gulonsäure fällt in der Regel bei der Kristallisation aus Wasser oder wasserhaltigen, organischen Lösungsmitteln an. Durch Abschleudern der Kristallmaische ist feuchtes Monohydrat zugänglich. Dieses kann als schleuderfeuchtes Produkt direkt in der nachfolgenden Veresterungsreaktion eingesetzt oder unter milden Bedingungen getrocknet werden.

Es ist auch möglich, eine aufkonzentrierte wäßrige Lösung der 2-Keto-L-gulonsäure direkt in die Veresterungsreaktion einzusetzen. Das überschüssige Lösungsmittel wird vor oder während der Veresterungsreaktion z.B. durch Extraktion und Phasentrennung oder Azeotropdestillation entfernt. Insbesondere eignet sich diese Vorgehensweise für eine Ketogulonsäure-Lösung aus einem fermentativen Herstellprozeß. Nach Abtrennung der Biomasse über Standard-Verfahren wie Filtration, Zentrifugation oder Fällung kann die meist gefärbte Fermentationslösung, vorzugsweise nach flüssig-flüssig Extraktion, ohne weitere Reinigung direkt eingesetzt werden. Das überschüssige Lösungsmittel wird danach, wie oben beschrieben, vor oder während der Veresterungsreaktion z.B. durch Phasentrennung oder Azeotropdestillation entfernt.

Wasserfreie 2-Keto-L-gulonsäure erhält man u.a. aus dem kristallinen, ggf. schleuderfeuchten Monohydrat durch Trocknung.

Vorteilhafterweise kann man bei dem erfindungsgemäßen Verfahren auf die Trocknung bzw. Entwässerung des Monohydrates der 2-Keto-L-gulonsäure verzichten, da bei der nachfolgenden, erfindungsgemäßen Aktivierungsreaktion ohnehin eine azeotrope Entwässerung durchgeführt wird. Durch Zusatz einer 0,005 bis 0,1 molaren, vorzugsweise einer 0,005 bis 0,05 molaren Menge eines sauren Katalysators, in freier oder polymer gebundener Form (als stark saurer Kationenaustauscher) oder ihres Esters wird die Veresterungsreaktion katalysiert. Unter der Bezeichnung "saurer Kationenaustauscher" sind kommerziell erhältliche Harze zu verstehen, wie z.B. Lewatit® S 100 und SP 112 (Bayer) oder Amberlite® 18 und IRA 120 oder Amberlyst® 15 oder Duolite® C 20, C 26 und C 264 (Rohm & Haas) oder Dowex® Ionentauscher. Auch klassische Zeolithe sind als Katalysator geeignet.

Als weitere Katalysatoren sind auch organische Säuren oder Mineralsäuren bzw. deren Derivate geeignet. Dazu zählen beispielsweise Phosphorsäure, Phosphorsäure-monobutylester, Phosphorsäuredibutylester, Phosphorsäure-monopentylester, Phosphorsäure-dipentylester, Schwefelsäure, Schwefelsäure-monobutylester, Schwefelsäure-monopentylester, Chlorwasserstoff, p-Toluolsulfonsäure, Methansulfonsäure, Chlorsulfonsäure, Trifluoressigsäure und andere starke, wasserfreie Säuren.

Auch 2-Keto-L-gulonsäure, 2,3:4,6-Di-O-isopropyliden-2-keto-L-gulonsäure oder L-Ascorbinsäure können als saure Veresterungskatalysatoren eingesetzt werden.

Bevorzugt verwendet man allerdings Schwefelsäure, Methansulfonsäure oder Monoalkylsulfate der verwendeten C₃-C₆-Alkohole, besonders bevorzugt sei Schwefelsäure genannt. Die Monoalkylsulfate spalten bei Temperaturen oberhalb von 70°C Schwefelsäure ab [Popelier, Bull. Soc. Chim. Belg. 35, 265 (1926)], die katalytisch wirkt. Daher ist bei Einsatz dieser Katalysatoren eine Veresterung meist erst bei höheren Temperaturen möglich.

Um einen möglichst vollständigen Umsatz bei der Veresterung zu erzielen, ist es von Vorteil, das Reaktionswasser möglichst vollständig zu entfernen. Vorteilhafterweise wird im vorliegenden Verfahren das Reaktionswasser mit überschüssigem Alkohol abdestilliert. Dies erfolgt in einem Druckbereich von 20 mbar bis Normaldruck, vorzugsweise in einem Bereich von 100 bis 800 mbar. Der Alkohol dient dabei als Schleppmittel für das gebildete Reaktionswasser. Alkohole mit weniger als 3 Kohlenstoffatomen sind dafür nicht so gut geeignet, aber prinzipiell möglich. Das Destillat wird nach der Entfernung des Wassers durch Phasentrennung, destillative Trocknung oder Trocknung durch wasserentziehende Mittel, wie z.B. Molekularsiebe, für weitere Veresterungsreaktionen verwendet.

Als Lösungsmittel zur Wasserauskreisung verwendet man vorteilhafterweise den Veresterungsalkohol oder eine Mischung aus diesem Alkohol und einem weiteren mit Wasser nicht mischbaren Lösungsmittel. Unter mit Wasser nicht mischbarem Lösungsmittel sind Lösungsmittel zu verstehen, die sich mit weniger als 2 Gew-% in Wasser lösen. Die erfindungsgemäß zu verwendenden Alkohole haben nur eine begrenzte Kapazität zur Ausschleusung des Reaktionswassers. Insbesondere bei hohen Katalysatorsäure-Konzentrationen oder kurzen Veresterungszeiten ist es von Vorteil, wenn bereits bei der Veresterungsreaktion ein zweites Lösungsmittel als Wasserschlepper zugegeben wird. Dieses Lösungsmittel sollte ein niedrig siedendes Azeotrop mit Wasser bilden und ggf. mit dem Alkohol nur begrenzt mischbar sein, um so den Alkohol während der Veresterungsreaktion nach Phasentrennung rückführen zu können. Von diesem zweiten Lösungsmittel werden nur geringe Mengen, vorteilhafterweise 10 bis 50 mol-% bezogen auf 2-Keto-L-gulonsäure, benötigt.

Vorteilhafterweise werden dafür unpolare halogenierte Kohlenwasserstoffe, wie z.B. Tetrachlorkohlenstoff, Chloroform, Dichlorethan, 1,2-Trichlorethylen, Perchlorethylen oder aromatische Kohlenwasserstoffe, wie Toluol, Xylol eingesetzt. Des weiteren kann auch Propylencarbonat verwendet werden. Als bevorzugtes Lösungsmittel sei Perchlorethylen genannt. In einer weiteren vorteilhaften Fahrweise werden C₁-C₆-Alkylhalogenide, die sich von Alkoholen, die nur eine Hydroxygruppe haben, ableiten, verwendet.

Der Umsatzgrad der 2-Keto-L-gulonsäure bei der Veresterungsreaktion liegt im erfindungsgemäßen Verfahren deutlich über 90 %, vorzugsweise in einem Bereich von 95 bis 99 %.

Im Verlauf der Veresterungsreaktion geht die 2-Keto-L-gulonsäure in Lösung, womit man einen guten optischen Indikator für den Fortschritt der Reaktion hat. In Abhängigkeit von der Menge der eingesetzten Katalysatorsäure erfolgt dies in einem Zeitraum von wenigen Minuten bis mehreren Stunden. Höhere Temperaturen, z.B. im Bereich von 30 bis 150°C begünstigen den Umsatz bei der Veresterungsreaktion. Gegen Ende der Reaktion, wenn ein Großteil des überschüssigen Alkohols abdestilliert worden ist, wird das Reaktionsgemisch zähflüssiger. Vorteilhafterweise kann die Veresterungsreaktion als abgeschlossen betrachtet werden, wenn eine lösungsmittel- und wasserfreie Schmelze des entsprechenden Keto-L-gulonsäure-alkylesters entstanden ist. Die Viskosität dieser Schmelze ist von den Substanzeigenschaften des jeweiligen 2-Keto-L-gulonsäureesters und der Temperatur abhängig.

Das Reaktionsgemisch erstarrt beim Abkühlen in einem Temperaturbereich von 20 bis 40°C. Beim Erwärmen bildet sich die Schmelze reversibel und ohne Zersetzung zurück. Ein erneuter Zusatz von trockenem Alkohol zur Vervollständigung der Veresterungsreaktion, wie beispielsweise in WO 99-3853 beschrieben, ist unter den oben genannten Bedingungen nicht erforderlich, weil a) die Veresterungsreaktion fast vollständig erfolgt und b) ein 100%iger Umsatz gemäß der Lehre der vorliegenden Erfindung nicht zwingend erforderlich ist. Bei Verwendung des Monohydrates der Keto-L-gulonsäure ist keine höhere Alkoholmenge zu Beginn der Veresterung erforderlich. Die Veresterungsgeschwindigkeit wird durch den Restwassergehalt des zurückgeführten Alkohols bestimmt.

Anstelle von 2-Keto-L-gulonsäure kann auch 2,3:4,6-Di-O-isopropyliden-2-keto-L-gulonsäure unter den oben genannten Bedingungen in gleicher Weise verestert werden. Dabei erfolgt zusätzlich eine Abspaltung der Aceton-Schutzgruppen. Für die Abspaltung werden zwei Moläquivalente Wasser benötigt, während gleichzeitig ein Moläquivalent Wasser bei der Veresterungsreaktion gebildet wird. Dies bedeutet, daß anstelle von wasserentziehenden Mitteln und physikalischen Trocknungsverfahren auch chemische Reaktionen zur Entfernung des Reaktionswassers eingesetzt werden können. In einfachster Weise setzt man daher das Monohydrat der 2,3:4,6-Di-O-isopropyliden-2-keto-L-gulonsäure ein. Die Reaktion erfolgt ebenfalls im oben beschriebenen Druckund Temperaturbereich.

Das gebildete Aceton wird als Leichtsieder während der Veresterungsreaktion zu Beginn bzw. zusammen mit überschüssigem, wasserhaltigem Lösungsmittel abdestilliert und kann nach Isolierung und Reingewinnung zurückgeführt werden.

Die Veresterungsreaktion von 2-Keto-L-gulonsäure bzw. 2,3:4,6-Di-O-isopropyliden-2-keto-L-gulonsäure kann diskontinuierlich oder kontinuierlich betrieben werden. Bei der kontinuierlichen Veresterung wird die Azeotropdestillation beispielsweise in einer Rührkesselkaskade, einem Dünnfilmverdampfer oder in ähnlich arbeitenden Apparaten durchgeführt. Auch unter diesen Bedingungen entsteht am Ende der Veresterung eine schmelzflüssige Form des entsprechenden 2-Keto-L-gulonsäure-alkylesters. Der Vorteil dieser Arbeitsweise besteht beispielsweise im Vergleich zur diskontinuierlichen Veresterung darin, daß die Reaktionszeit bei gleichem Umsatz und gleicher Reinheit deutlich unter einer Stunde liegt.

In der bevorzugten Ausführungsform des erfindungsgemäßen Verfahrens erhält man nach den Verfahrensschritten a) und b) eine Schmelze bzw. schmelzflüssige Form des Esters, die gut fließfähig ist und leicht in Rohrleitungen transportiert werden kann. Diese Schmelze kann ohne Isolierung bzw. ohne weitere Reinigung direkt unter 100°C bei Normaldruck unter Säurekatalyse in Gegenwart ausschließlich eines Lösungsmittels oder Lösungsmittelgemisches lactonisiert werden, das mit Wasser mischbar ist und in situ ein Lösungsmittel bildet, in dem die gebildete Ascorbinsäure schwer löslich ist, dabei wird der zur Aktivierung der verwendete Alkohol freigesetzt und die 2-Keto-L-gulonsäure zu L-Ascorbinsäure hoher Reinheit umgelagert.

Im Anschluß an die Veresterungsreaktion der 2-Keto-L-gulonsäure bzw. 2,3:4,6-Di-O-isopropyliden-2-keto-L-gulonsäure erfolgt im Verfahrensschritt c) die Umlagerung (Lactonisierung) des gebildeten Esters - in Form seiner Schmelze - zu L-Ascorbinsäure in Gegenwart eines sauren Katalysators. Bevorzugt handelt es sich dabei um eine wasserfreie Schmelze. Die Lactonisierung nach dem beanspruchten Verfahren kann auch ausgehend von der freien 2-Keto-L-gulonsäure bzw. 2,3:4,6-Di-O-isopropyliden-2-keto-L-gulonsäure erfolgen. Dies führt jedoch in der Regel zu geringfügigen Ausbeuteverlusten.

Gegenstand der vorliegenden Erfindung ist eine Lactonisierung von 2-Keto-L-gulonsäure, 2,3:4,6-Di-O-isopropyliden-2-keto-L-gulonsäure oder deren C₃-C₆-Alkylester in Abwesenheit eines inerten Lösungsmittels, wie unpolare halogenierte Kohlenwasserstoffe, wie z.B. Tetrachlorkohlenstoff, Chloroform, Dichlorethan, 1,2-Trichlorethylen oder Perchlorethylen oder aromatische Kohlenwasserstoffe, wie Toluol, Benzol oder Xylol; jedoch in Gegenwart ausschließlich eines Lösungsmittels oder Lösungsmittelgemisches, das mit Wasser mischbar ist und in situ ein Lösungsmittel bildet, in dem die gebildete Ascorbinsäure schwer löslich ist. Unter mit Wasser mischbaren Lösungsmitteln bzw. Lösungsmittelgemischen sind Lösungmittel oder deren Gemische zu verstehen, die bei Raumtemperatur (= 23 °C) mindestens 5 Gew-%, vorteilhaft mindestens 7 Gew-%, bevorzugt mindestens 10 Gew-%, ganz besonders bevorzugt mindestens 20 Gew-% Wasser enthalten. Unter für Ascorbinsäure schwer löslich ist eine Löslichkeit von weniger als 2 g/l, bevorzugt von weniger als 1 g/l im Lösungsmittel bei Raumtemperatur zu verstehen. Das Lösungsmittel oder Lösungsmittelgemisch wird zu Beginn der Lactonisierungsreaktion zugesetzt und/oder ist noch aus der Veresterungsreaktion nach unvollständiger Destillation des Lösungsmittels noch vorhanden. Es kann falls erforderlich während der Reaktion diskontinuierlich oder kontinuierlich nachgegeben werden. Aus diesem Lösungsmittel oder Lösungsmittelgemisch bildet sich in situ ein weiteres Lösungsmittel, in dem Ascorbinsäure nur schwer löslich ist. Dieses weitere Lösungsmittel kann der Lactonisierungsreaktion vorteilhaft schon von Beginn der Reaktion zugesetzt werden und gegebenenfalls während der Reaktion diskontinuierlich oder kontinuierlich zugesetzt werden.

Als Lösungsmittel oder Lösungsmittelgemische eignen sich vorteilhaft C₁-C₆-Alkohole, die nur eine Hydroxygruppe tragen, oder deren Mischungen. Es können primäre, sekundäre oder tertiäre Alkohole oder deren Mischungen verwendet werden, primäre oder sekundäre Alkohole sind bevorzugt. Vorteilhafter Weise werden die gleichen erfindungsgemäßen höheren Alkohole, wie sie zur Veresterung der Keto-gulonsäuren eingesetzt werden, verwendet. Dies sind insbesondere Alkohole mit einer Kohlenstoffanzahl größer gleich 3, wie z.B. n-Propanol, Isopropanol, n-Butanol, iso-Butanol, tert-Butanol, 1-Pentanol, 2-Pentanol, 3-Pentanol, 1-Hexanol, 2-Hexanol, 1-Heptanol oder 2-Heptanol. Bevorzugt wird n-Butanol, iso-Butanol oder tert-Butanol verwendet. Besonders bevorzugt sind die reinen Alkohole der vorgenannten C₁-C₆-Alkohole, da sie leicht abgetrennt werden können. Die vorgenannten Alkohole bilden in Gegenwart von beispielsweise Salzsäure oder Chlorwasserstoff die entsprechenden Chloralkylhalogenide, in denen Ascorbinsäure nur schwer löslich ist. Diese Chloralkylhalogenide in Kombination mit den Ausgangsalkoholen ermöglichen eine leichte und einfache Reinigung der entstehenden Ascorbinsäure und können kostengünstig und einfach in den Prozeß rückgeführt werden. Die so in situ in geringen Mengen gebildeten mono-Alkylhalogenide vereinfachen die Reinigung des Lactonisierungsprodukts Ascorbinsäure. Falls erforderlich können diese Alkylhalogenide der Reaktion vor und während der Lactonisierung diskontinuierlich oder kontinuierlich zugegeben werden. Vorteilhafte Alkylhalogenide sind C₁-C₆-Alkylhalogenide ausgewählt aus der Gruppe Ethylchlorid, Propylchlorid, Butylchlorid, tert.-Butylchlorid, Pentylchlorid oder Hexychlorid. Besonders bevorzugt sind Butylchlorid, tert.-Butylchlorid oder Pentylchlorid.

Bei dem erfindungsgemäßen Verfahren wird die Umlagerung vorteilhafterweise mit einem Ester, z.B. mit dem Butylester der 2-Keto-L-gulonsäure oder der 2,3:4,6-Di-O-isopropyliden-2-keto-L-gulonsäure durchgeführt. Die Lactonisierung kann wie oben beschrieben aber auch mit geringfügigen Ausbeuteverlusten in Gegenwart der freien Säure durchgeführt werden.

Bei Verwendung des Butylesters für die Lactonisierung und Vorlage der oben genannten Alkohole vorzugsweise in Form des Butanols bildet sich aus Butanol und Chlorwasserstoff und/oder wäßriger Salzsäure in geringen Mengen Butylchlorid. Dieses beeinflußt die Lactonisierungsreaktion vorteilhaft. Insbesondere wirkt sich dies bei der bevorzugten kontinuierlichen Reaktionsführung in einem Rohrreaktor vorteilhaft aus, da das zusätzlich entstehende Lösungsmittel für eine weitere Verringerung der Löslichkeit des Wertproduktes sorgt. Damit kann mit einer höheren Konzentration des Gulonsäureesters beispielsweise des 2-Keto-L-gulonsäurebutylesters in den Rohrreaktor hineingefahren werden. Die Raum-Zeit-Ausbeute verbessert sich signifikant. Als zusätzlicher Vorteil bietet dieses Lösungsmittels neben zum Stand der Technik vergleichbaren oder besseren hohe Ausbeuten und Reinheiten auch die Möglichkeit einer gleichzeitigen einfachen Rückführungsmöglichkeit des Lösungsmittels. Geringfügige Verluste des Lösungsmittels im Zuge der Aufarbeitung des Rohproduktes werden durch die gewünschte Nachbildung des Lösungsmittels während der Umlagerungsreaktion ausgeglichen. In einer vorteilhaften Fahrweise des Verfahrens können über die Zugabe der entsprechenden Alkylhalogenide wie der bevorzugten Chloralkylhalogenide die Verluste ausgeglichen werden oder aber es kann eine vorteilhafte höhere definierte Konzentration der Alkylhalogenide im Verfahren eingestellt werden.

Im Vergleich zum Stand der Technik, nach dem der für die Veresterung benötigte reine Alkohol beispielsweise n-Butanol oder n-Propanol nur durch eine mehrstufige, komplexe Destillation vom inerten, mit dem Alkohol ein Azeotop bildenden Lösungsmittel, z.B. Perchlorethylen zurückgewonnen werden kann, bedeutet dies eine signifikante Vereinfachung des Verfahrens. So bildet Butylchlorid mit Butanol unter den Bedingungen der Lösungsmittelaufarbeitung kein Azeotrop.

Das erfindungsgemäße Verfahren der Lactonisierung sowie der vorangehenden Veresterung kann deshalb vorteilhaft kontinuierlich durchgeführt werden. Es können auch einzelne Verfahrensstufen kontinuierlich durchgeführt werden. Beispielsweise erfolgt die Lactonisierung des Butylesters nach schnellem Erhitzen auf Reaktionstemperatur in einem Rohrreaktor vorteilhaft in einem Gemisch aus n-Butanol/Butylchlorid/HCl und Wasser.

Durch die Verwendung der genannten Lösungsmittel kann die als Katalysator eingesetzte vorteilhafte Salzsäure oder Chlorwasserstoff direkt nach der Reaktion durch spontane Entspannung in ein Verdampfersystem hinein mit einem Teil des eingesetzten Lösungsmittels bzw. Lösungsmittelgemisches zurückgeführt werden. Die üblicherweise erforderliche aufwendige Aufkonzentration der anfallenden verdünnten Katalysatorsäure vor der Rückführung kann vollständig entfällen. Auch der in der Regel vorhandene zusätzlich installierte Ionenaustauscher zur Neutralisation der Mutterlauge kann entfallen.

Ein weiterer Vorteil des erfindungsgemäßen Verfahrens besteht darin, daß im Gegensatz zu den bekannten Verfahren die farbgebenden Komponenten und andere unerwünschten Nebenprodukte nach der Extraktion der Butanolphase mit Wasser in der Butanolphase verbleiben und durch einfache Verdampfung als Sumpfprodukt ausgeschleust werden können.

Durch diese Maßnahmen kann bei dem erfindungsgemäßen Verfahren weitgehend auf eine Entfernung der gefärbten und unerwünschten Nebenprodukte durch ein Aktivkohlebett verzichtet werden.

Das Verfahren zur kontinuierlichen Lactonisierung läßt sich, wie oben beschrieben, vorteilhaft mit der kontinuierlichen Veresterung der Keto-gulonsäuren kombinieren. Die im Verfahren anfallenden alkoholischen Lösungen, beispielsweise die butanolische Lösung bzw. Schmelze, kann direkt der kontinuierlichen Lactonisierung unterworfen werden.

Für die Umsetzung des erfindungsgemäßen Verfahrens eignen sich besonders 2-Ketö-L-gulonsäureester oder Diaceton-2-keto-L-gulonsäureester. Die Herstellung der Ester ist nicht auf das oben beschriebene Verfahren beschränkt, sondern dient hier lediglich zur Veranschaulichung der vorteilhaften Arbeitsweise. So ist die Herstellung der Ester beispielsweise auch aus einer wäßrigen Fermentationslösung, gegebenenfalls durch Einbindung klassischer Aufreinigungsverfahren (Ionentausch, Aktivkohleadsorption oder ähnlichen Verfahren), durch extraktive Veresterung oder Direktveresterung in einer Reaktionskolonne in guten Ausbeuten realisierbar.

Die Umlagerung (= Lactonisierung) erfolgt nach dem erfindungsgemäßen Verfahren bei Temperaturen über 60°C, vorteilhaft in einem Temperaturbereich von 70 bis 150°C, bevorzugt zwischen 70 und 130°C, besonders bevorzugt zwischen 75 bis 110°C. Wird das Verfahren kontinuierlich ausgeübt, so ist die Temperatur vorteilhaft über 90°C zu halten. Die Reaktionszeit wird der entsprechenden Umlagerungstemperatur in dem Fachmann bekannter Weise angepaßt. Der Druck der Reaktion liegt vorteilhaft zwischen 1 und 20 bar, vorteilhaft zwischen 1 und 15 bar, bevorzugt zwischen 1 und 10, besonders vorteilhaft zwischen 2 und 6 bar. Die Lactonisierung kann auch bei vermindertem Druck durchgeführt werden, dies ist jedoch nicht sinnvoll. Die Reaktionszeiten liegen im vorgenannten Temperaturbereich bei Normaldruck vorzugsweise zwischen 0,25 und 25 Stunden. Unter diesen Bedingungen werden hohe Ausbeuten und hohe Reinheiten der L-Ascorbinsäure erreicht.

Für den Erfolg der Umlagerungsreaktion ist die eingesetzte Katalysatorsäure von entscheidender Bedeutung. Mineralsäuren, wie z.B. Phosphorsäure oder Schwefelsäure sind geeignet, liefern aber niedrige Ausbeuten an Wertprodukt. Vorteilhafterweise setzt man Salzsäure, entweder als konzentrierte wäßrige Salzsäure oder als Gas, das direkt in das Umlagerungsgemisch eingeleitet wird, ein. Für die Umlagerung wird Wasser benötigt. Daher muß bei Einsatz von Chlorwasserstoff die erforderliche Menge Wasser zugesetzt werden. Eine zu hohe Säurekonzentration bedingt in Gegenwart von Wasser geringere Ausbeuten an Wertprodukt und schlechtere Reinheiten. Eine Konzentration von 0,5 bis 10%, bevorzugt 0,75 bis 7,5%, besonders bevorzugt von 1 bis 5 % bezogen auf Chlorwasserstoffgas in der oben angegebenen Mischung aus höherem Alkohol und Lösungsmittel wird bevorzugt eingesetzt.

Die Katalysatorsäure kann im Umlagerungsreaktor mit dem inerten Lösungsmittel vorgelegt, in das Lösungsmittel bei Temperaturen bis 50°C eingegast oder als wäßrige Lösung zu dem Umlagerungsgemisch zugegeben werden.

Für längere Umlagerungszeiten bei erfindungsgemäß niedrigeren Temperaturen läßt man in der Regel bevorzugt die schmelzflüssige Form beispielsweise des 2-Keto-L-gulonsäure-alkylesters, vorzugsweise des Butylesters, in das vorgelegte Lösungsmittel zusammen mit der Katalysatorsäure laufen oder man arbeitet invers. In allen Fällen ist eine ausreichende Durchmischung der Säure mit dem Reaktionsgemisch für den Erfolg der Umlagerung wichtig. Die gebildetet L-Ascorbinsäure fällt gegen Ende der Umlagerung in kristalliner Form aus und kann durch übliche Verfahren, wie z.B. Absaugen, Abschleudern, Abpressen oder Extraktion isoliert werden. Nach dem Waschen mit dem Alkohol, der auch für die Veresterung eingesetzt wurde, und Trocknen erhält man das Vitamin C als Rohprodukt in hohen Reinheiten von mindestens 95%, bevorzugt mindestens 97%, besonders bevorzugt mindestens 98%, ganz besonders bevorzugt mindestens 99% chemischer Reinheit und guten Ausbeuten von mindestens 80%, bevorzugt mindestens 85%, besonders bevorzugt von mindestens 87%.

Durch das vorteilhafte erfindungsgemäße Verfahren kann die Reinheit und Ausbeute des Reaktionsproduktes signifikant verbessert werden. Bei der Umlagerung werden vorteilhafterweise zusätzlich in situ gezielt geringe Mengen von aktiviertem Kohlenstoff in fein verteilter Form erzeugt, wodurch Spuren unerwünschter Nebenprodukte adsorbiert und mechanisch leicht abgetrennt werden können. Damit wird eine aufwendige Reinigung der entstandenen L-Ascorbinsäure überflüssig. Der Anteil an aktiviertem Kohlenstoff liegt im Bereich von 0,1 bis maximal 0,8 %. Diese Mengen reichen aus, um beispielsweise gefärbte Verunreinigungen, die ggf. aus fermentativ hergestellter 2-Keto-L-gulonsäure stammen können, wirksam zu adsorbieren. Der gebildete Kohlenstoff kann im Zuge der Isolierung der Ascorbinsäure bequem abgetrennt werden.

Die Ausbeute und Reinheit an Vitamin C wird durch die Zusammensetzung des Lösungsmittelgemisches im Verlauf der Umlagerung beeinflußt. Eine zu hohe Wasserkonzentration reduziert die Ausbeute. Der Wassergehalt in dieser Mischung sollte daher im Bereich von 1 bis 10%, vorzugsweise im Bereich von 2 bis 7% liegen. Gegen Ende der Umlagerung enthält das Umlagerungsgemisch vorteilhafterweise 50 bis 90 Gewichtsprozent des Lösungsmittels und 50 bis 10 Gewichtsprozent des gebildeten Alkohols. In dieser Mischung sind L-Ascorbinsäure schwer und alle anderen Edukte bzw. Nebenprodukte und die Katalysatorsäure leicht löslich.

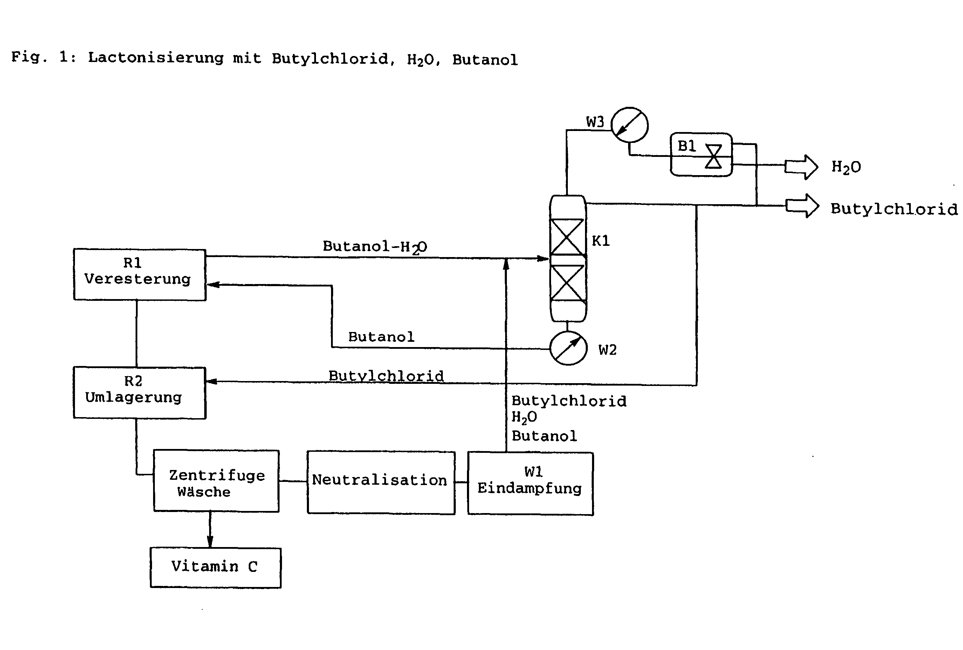

Die Rückführung und destillative Aufarbeitung des Lösungsmittel bzw. Lösungsmittelgemisches erfolgt vorteilhafterweise nach dem in Figur 1 dargestellten Verfahren.

Figur 1 zeigt beispielhaft die Veresterung der 2-Keto-L-gulonsäure mit Butanol. Das bei der Veresterungsreaktion gebildete Lösungsmittelgemisch Butanol - Wasser wird der Lösungsmittelaufarbeitung zugeführt. In diesen Produktstrom wird zusätzlich die bei der Veresterungsreaktion anfallende Mutterlauge, enthaltend Butylchlorid, Butanol und Wasser (ggf. Schwersieder) eingespeist. Die Trennung des Azeotrops Butylchlorid / Wasser kann durch eine einfache Zweidruck-Destillation oder durch eine Extraktivdestillation in den nach dem Stand der Technik bekannten Apparaten erreicht werden. Bei Verwendung nach dem Stand der Technik anderer halogenierter Lösungsmittel, wie z.B. Perchlorethylen wäre zusätzlich die Trennung des Azeotrops Butanol / Perchlorethylen erforderlich. Diese ist sehr aufwendig.

Unter den gleichen Bedingungen, unter denen die 2-Keto-L-gulonsäure-alkylester umgelagert werden können, läßt sich auch das Veresterungsprodukt ausgehend von Diaceton-2-keto-L-gulonsäure umsetzen. Die Ausbeuten und Reinheiten sind vergleichbar.

Das erfindungsgemäße Verfahren wird bevorzugt kontinuierlich durchgeführt. Obwohl auch eine Reihe anderer saurer Katalysatoren, wie z.B. Mineralsäuren, geeignet sind, so wird auch bei der kontinuierlich geführten Umlagerung vorzugsweise in Gegenwart des azeotrop-bildenden Katalysators Chlorwasserstoff gearbeitet. Dessen Effektivität läßt sich unterstützen bzw. steigern, wenn ein Katalysator auf fester Phase, z.B. in Gegenwart eines halogenhaltigen Zeoliths, wie er in EP 988891 beschrieben ist, gearbeitet wird. Katalysatoren dieses Typ zeichnen sich durch u.a. durch hohe Chloridkonzentrationen an der Oberfläche und überraschend lange Standzeiten aus.

Im Gegensatz zur diskontinuierlichen Verfahrensweise wird die kontinuierliche Reaktion unter Druck ausgeführt.

Je nach Einsatzstoff liegt die Verweilzeit im Rohrreaktor in der Größenordnung von 1 min bis 1 Stunde. Bei Verwendung des 2-Keto-L-gulonsäurebutylesters sind Verweilzeiten in der Größenodnung von 5 bis 20 Minuten ausreichend. Die Zusammensetzung des Eduktes ist für die Verweilzeit wichtig und daher vom Fachmann den jeweiligen Gegebenheiten nach üblichen Kriterien anzupassen. Die Verweilzeit wird so gewählt, daß die Strömung im Reaktor einer Pfropfenströmung nahe kommt.

Die alkoholische Esterlösung wie die butanolische Butylesterlösung und ein Gemisch aus Alkohol/Alkylhalogenid/Chlorwasserstoff/ Wasser wie n-Butanol / Butylchlorid / Chlorwasserstoff und Wasser werden nach schnellem Erhitzen auf Reaktionstemperatur über einen Mischer dem Rohrreaktor zugeführt. Lange Verweilzeiten während der Aufheizphase sind zu vermeiden, da sonst unerwünschte Folgereaktionen eintreten können. Zum schnellen Aufheizen eignen sich gängige Wärmetauscher hoher Leistung, Induktionsschleifen oder handelsübliche Mikrowellen-Durchlauferhitzer, z.B. µWaveFlow 2541 (Fa.Püschner).

Nach Ende der Reaktion wird das Reaktionsgemisch schlagartig in einen unter Normaldruck arbeitenden Verdampfer entspannt und ein Teil des Lösungsmittel verdampft. Zur Entspannung und Nachverdampfung eignen sich insbesondere Fallstromverdampfer.

Dadurch wird aus dem flüssigen Reaktionsaustrag ein Produktgemisch bestehend aus n-Butanol / Butylchlorid / HCl und Wasser entnommen und einem nachgeschalteten Kondensator zugeführt.

Die Aufarbeitung dieses Produktgemisches erfolgt in üblicher und sehr einfacher Weise durch Kondensation bei Normaldruck. Nach der Kondensation wird das Gemisch mit der Hauptmenge an Chlorwasserstoff durch Förderung, z.B. mit einer Kolben- oder Membranpumpe, direkt in die Umlagerung zurückgeführt.

Im Sumpf des Verdampfers fällt eine nahezu Chlorwasserstoff-freie Roh-Ascorbinsäure in alkoholischer Lösung beispielsweise in n-Butanol an.

Zur Vorreinigung wird der Reaktionsaustrag einer Extraktion mit Wasser unterzogen. Diese kann nach dem Stand der Technik in einem kontinuierlich arbeitenden Mixer-Settler-Apparat oder in einer Extraktionskolonne erfolgen.

Die farbgebenden Komponenten und andere unerwünschte Nebenprodukte verbleiben in der organischen Butanolphase. Die so gewonnene Ascorbinsäure findet sich in der fast farblosen Wasserphase und hat eine überraschend hohe Reinheit. Sie liegt in der Größenordnung von 98,5-99,5%.

Nach Abtrennung des Restbutanols aus der wässrigen Phase durch Rektifikation wird die Mutterlauge als Sumpfprodukt direkt der Kristallisation zugeführt.

Vorteilhafte Ausführungsformen sind in den nachstehend beschriebenen Ausführungsbeispielen beschrieben.

### Beispiele:

### Ausführungsbeispiele für die diskontinuierliche Fahrweise

Die Ausbeuten und Reinheiten der beschriebenen Versuche sind nicht optimiert und beziehen sich auf isoliertes, getrocknetes Vitamin C. Die Ausbeuteangaben basieren auf Mol %. Die Reinheit der 2-Keto-L-gulonsäure und der Diaceton-2-keto-L-gulonsäure wurde durch HPLC kalibriert gegen eine Referenzprobe gemessen. Der Gehalt an Vitamin C wurde im Rohprodukt mit der üblichen iodometrischen Titrationsmethode ermittelt.

### Herstellung von 2-Keto-L-gulonsäure-butylester in Butanol

### Beispiel 1

Der als Edukt für die diskontinuierliche oder kontinuierliche Umlagerung eingesetzte Butylester wurde in einer kontinuierlich arbeitenden Veresterungskolonne hergestellt.

Die Versuchskolonne hatte einen Durchmesser von 30mm. Die Kolonne war über eine Höhe von 3m mit Blechpackungen bestückt. Die theoretische Trennstufenzahl der Kolonne lag bei insgesamt 18 Trennstufen. Der Zulauf der wässrigen Ketogulonsäure war auf der 12ten theoretischen Trennstufe (von unten her gezählt) angebracht. Der Zulauf für n-Butanol und für die katalytische Menge an Schwefelsaure befand sich am Kopf der Kolonne.

Bei einem Kopfdruck von 580 mbar wurden 100 g/h 50% wässrige 2-Keto-L-gulonsäurelösung auf die 12. theoretische Trennstufe zugefahren. Am Kopf der Kolonne wurden 173 g/h n-Butanol zusammen mit 0.032 mol Schwefelsäure (98%ig) pro mol Ketogulonsäure zugefahren. Es stellte sich eine Sumpftemperatur von 103°C ein. Das Azeotrop Wasser/n-Butanol wurde am Kopf der Kolonne kondensiert und in einen Abscheider geleitet. Die untere wässrige Phase wurde entnommen und entsorgt. Die obere organische Phase wurde wieder auf den Kopf der Kolonne aufgegeben. Aus dem Sumpf der Kolonne wurden 63,5 g/h KGS als 30% butanolische Lösung entnommen. Dies entspricht einer durchschnittlichen Ausbeute von 98,8 %.

### Beispiel 2

Das Reaktionsgemisch aus der kontinuierlich betriebenen Veresterung von 594 g (3 mol) wasserfreier 2-Keto-L-gulonsäure und 329 ml (3,6 mol) n-Butanol wurde in 730 ml (7 mol) 1-Butylchlorid mit 79 g (0,8 mol) konz. Salzsäure versetzt und 17 Stunden bei 75°C nachgerührt. Die aus der dunkel gefärbten Reaktionsmischung ausgefallene L-Ascorbinsäure wurde abgesaugt, mit n-Butanol nachgewaschen und im Vakuum getrocknet.

Ausbeute: 458 g (86%) graues Kristallisat mit einer Vitamin C Reinheit von 99 %.

Durch literaturbekannte Verfahren, z.B. Umkristallisation konnte Vitamin C hoher Reinheit gewonnen werden.

### Beispiel 3

Gemäß Beispiel 2 wurde anstelle von wasserfreier 2-Keto-L-gulonsäure 2-Keto-L-gulonsäure Monohydrat eingesetzt. Die Ausbeute an L-Ascorbinsäure (Rohprodukt) betrug 85 % (Reinheit 98,3 %).

### Beispiel 4

Gemäß Beispiel 2 wurde anstelle von wasserfreier 2-Keto-L-gulonsäure eine 30 %ige wäßrige 2-Keto-L-gulonsäurelösung in die Veresterungsreaktion eingesetzt. Die Ausbeute an L-Ascorbinsäure (Rohprodukt) betrug 85,3 % (Reinheit 98,5 %).

### Beispiel 5

In einem Lösungsmittelgemisch bestehend aus 730 ml (7 mol) 1-Butylchlorid und 329 ml (3,6 mol) n-Butanol wurden 594 g (3 mol) 2-Keto-L-gulonsäure suspendiert, auf 73°C aufgeheizt und nach Zusatz von 79 g (0,8 mol) konzentrierter Salzsäure 20 Stunden nachgerührt. Die ausgefallenen, schwarzgrau gefärbten Kristalle wurden abgesaugt, mit Butanol nachgewaschen und im Vakuum getrocknet. Ausbeute: 79 %. Die Reinheit des Produktes betrug nur 97,5%.

### Beispiel 6

In Analogie zu Beispiel 1 und 2 wurde verestert und umgelagert. Der Unterschied bestand aber darin, daß das goldgelbe Veresterungsgemisch in Butylchlorid / Salzsäure eingerührt wurde. Die Ausbeute an getrocknetem Rohprodukt betrug 89 % (Reinheit 99,5 %).

### Beispiel 7

Gemäß Beispiel 1 und 2 wurde verestert und umgelagert. Die ausgefallene L-Ascorbinsäure wurde mit insgesamt 450 ml Wasser zweimal extrahiert und nach dem Klarfiltrieren aufgereinigt. In der wäßrigen Lösung befanden sich nach schonendem Eindampfen im Vakuum 87 % L-Ascorbinsäure mit einer Reinheit von 99 %.

### Beispiel 8

In 667 g (9 mol) n-Butanol wurden 584 g (2 mol) Diaceton-2-keto-L-gulonsäure Monohydrat suspendiert und nach Zusatz von 5 g konzentrierter Schwefelsäure auf 200 mbar evakuiert. Nach Erhitzen auf 85 °C wurden nach 2 Stunden 580 g aceton- und wasserhaltiges n-Butanol abdestilliert. Der viskose, goldgelb gefärbte Rückstand wurde mit 730 ml (7 mol) Butylchlorid versetzt und nach Zusatz von 84 ml konz. Salzsäure 17 Stunden bei 72-75°C umgelagert. Die ausgefallene L-Ascorbinsäure wurde abgesaugt, mit n-Butanol gewaschen und im Vakuum getrocknet. Man erhielt 580 g (87 %) eines hellgrauen Rohproduktes mit einer Reinheit von 98,9%.

### Ausführungsbeispiele für die kontinuierliche Fahrweise

In den folgenden Beispielen wird der Verbund mit der kontinuierlichen Veresterung von Keto-L-gulonsäure zu KGS-butylester beschrieben.

### Beispiel 9

Aus dem Sumpfkreislauf der Veresterungskolonne wurden ca. 1000 g/h 25%-ige butanolische 2-Keto-L-gulonsäurebutylester-Lösung über einen Wärmeaustauscher in die Reaktionspumpe geleitet. Das rückgeführte Lösungsmittelgemisch n-Butanol/Butylchlorid/HCl/Wasser wurde über den Wärmeaustauscher in die Reaktionspumpe geleitet. Die Ausgangstemperatur nach dem Wärmaustauscher wurde auf 120°C eingestellt. Der Druck betrug 3,9 bar. In der Reaktionspumpe und im Nachreaktor wurde die Temperatur bei 120°C gehalten.

Das Reaktionsgemisch wurde spontan in den Verdampfer entspannt.

Der Verdampfer wurde bei Normaldruck betrieben, daraus ergab sich eine Verdampfungstemperatur von 108°C. Im Kondensator wurden ca. 1950 g/h mit einer Zusammensetzung von 82% n-Butanol, 3% Butylchlorid, 8% und in den Reaktor zurückgeführt.

Im Sumpf des Verdampfers wurden ca. 1050 g/h mit einer Zusammensetzung von 82% n-Butanol, 0,3% Butylchlorid, 1000 ppm HCl, 1% Wasser und 16% Ascorbinsäure entnommen. Die Temperatur des Sumpfaustrags betrug 108°C. Der Sumpfaustrag wurde über den Kühler geleitet und auf eine Temperatur von 30°C abgekühlt.

Danach wurde das Sumpfprodukt des Verdampfers in einer 2-stufigen Extraktionsapparatur mit Wasser extrahiert. Die schwarz gefärbte Butanolphase wurde im Verdampfer von den farbgebenden Komponenten und anderen Nebenprodukten abgetrennt. Das wasserfeuchte Destillat wurde in die Veresterungskolonne zurückgeführt.

Die farblose Wasserphase hatte folgende Zusammensetzung:

| | |
|---|---|
| 30 % | Ascorbinsäure |
| 10 % | n-Butanol |
| 60 % | Wasser |
| <0,1 % | HCl |
| <0,1 % | Nebenprodukte |

Die wässrige Ascorbinsäurelösung wurde in einer Rektifizierkolonne vom n-Butanol befreit auf einen Wassergehalt von 20 bis 40 % eingestellt.

Für die Hochreinigung war eine einfache, zweistufige Kristallisation ausreichend.

Die Ausbeute an L-Ascorbinsäure gemäß der beschriebenen Verfahrensvariante lag zwischen 92% und 95%, bezogen auf den eingesetzten 2-Ketogulonsäurebutylester. Die Reinheit des Rohproduktes lag zwischen 98,5 und 99,5 %.

Durch eine nachgeschaltete Kristallisationsstufe kann nach dem Stand der Technik eine Hochreinigung der L-Ascorbinsäure erfolgen.

### Beispiel 10

Die Ausführungsform entsprach der des Beispiels 2. Der Unterschied bestand aber darin, daß der Rohrreaktor mit einem Katalysator gemäß EP 988891 gepackt war. Die Reaktionsbedingungen blieben unverändert.

Die Ausbeute an isoliertem Roh C lagen bei 93,5%. Das fast farblose Produkt hatte eine Reinheit von 99%. Eine Belegung bzw. ein Aktivitätsverlust des Katalysators wurde auch nach ca. 500 Zyklen nicht beobachtet.

## Patentansprüche

1. Verfahren zur Herstellung von L-Ascorbinsäure, **dadurch gekennzeichnet, daß** man 2-Keto-L-gulonsäure oder eine Schmelze von 2-Keto-L-gulonsäure-C₃-C₆-alkylester unter Säurekatalyse in Gegenwart ausschließlich eines Lösungsmittels oder Lösungsmittelgemisches lactonisiert, das mit Wasser mischbar ist und in situ ein Lösungsmittel bildet, in dem die gebildete Ascorbinsäure eine Löslichkeit von weniger als 2 g/l bei Raumtemperatur hat.

2. Verfahren nach Anspruch 1, **dadurch gekennzeichnet, daß** man die Lactonisierung unter Katalyse einer Mineralsäure durchführt.

3. Verfahren nach einem der Ansprüche 1 oder 2, **dadurch gekennzeichnet,daß** man die Lactonisierung mit Chlorwasserstoff oder wässriger Salzsäure katalysiert.

4. Verfahren nach den Ansprüchen 1 bis 3, **dadurch gekennzeichnet, daß** man die Lactonisierung in Gegenwart eines C₁-C₆-Alkohols als Lösungsmittel durchführt.

5. Verfahren nach den Ansprüchen 3 oder 4, **dadurch gekennzeichnet, daß** sich in situ aus dem Alkohol C₁-C₆-Alkylhalogenide bilden.

6. Verfahren nach den Ansprüchen 3 bis 5, **dadurch gekennzeichnet, daß** man zu Beginn oder während der Lactonisierung ein Alkylhalogenid eines C₁-C₆-Alkohols der Reaktion zusetzt.

7. Verfahren nach den Ansprüchen 3 bis 6, **dadurch gekennzeichnet, daß** man zu Beginn oder während der Lactonisierung ein Alkylhalogenid eines C₁-C₆-Alkohols ausgewählt aus der Gruppe Ethylchlorid, Propylchlorid, Butylchlorid, tert.-Butylchlorid, Pentylchlorid oder Hexylchlorid zusetzt.

8. Verfahren nach den Ansprüchen 1 bis 7, **dadurch gekennzeichnet, daß** man die Lactonisierung in einer kontinuierlichen Reaktion durchführt.

9. Verfahren zur Herstellung von L-Ascorbinsäure, **dadurch gekennzeichnet, daß** es folgende Schritte umfaßt:
a) Veresterung von 2-Keto-L-gulonsäure oder 2,3:4,6-Di-O-isopropyliden-2-keto-L-gulonsäure in Gegenwart eines sauren Katalysators mit einem C₃-C₆-Alkohol,
b) Destillation des überschüssigen C₃-C₆-Alkohols zusammen mit dem gebildeten Reaktionswasser und
c) Lactonisierung des gebildeten 2-Keto-L-gulonsäure-C₃-C₆-alkylester unter Säurekatalyse in Gegenwart ausschließlich eines Lösungsmittels oder Lösungsmittelgemisches, das mit Wasser mischbar ist und in situ ein Lösungsmittel bildet, in dem die gebildete Ascorbinsäure schwer löslich ist.

10. Verfahren nach Anspruch 9, **dadurch gekennzeichnet, daß** man die Veresterung im Verfahrensschritt a) mit einem Alkohol, ausgewählt aus der Gruppe n-Propanol, Isopropanol, 1-Butanol, 2-Butanol, 2-Methyl-1-propanol, 2-Methyl-2-propanol, 1-Pentanol oder 1-Hexanol durchführt.

11. Verfahren nach Anspruch 9 oder 10, **dadurch gekennzeichnet, daß** man die Veresterung im Verfahrensschritt a) in Gegenwart einer Mineralsäure, eines sauren Ionenaustauschers oder eines trägerfixierten Katalysators durchführt.

12. Verfahren nach den Ansprüchen 9 bis 11, **dadurch gekennzeichnet, daß** man die Veresterung im Verfahrensschritt a) mit n-Butanol in Gegenwart von Schwefelsäure durchführt.

13. Verfahren nach den Ansprüchen 9 bis 12, **dadurch gekennzeichnet, daß** die Reaktionstemperaturen in den Verfahrensschritten a) bis c) im Bereich von -10 bis 160°C liegen.

14. Verfahren nach den Ansprüchen 9 bis 13, **dadurch gekennzeichnet, daß** die Veresterung und Lactonisierung in den Verfahrensschritten a) bis c) bei Drücken im Bereich von 0,1 bis 20 bar erfolgen.

15. Verfahren nach den Ansprüchen 9 bis 14, **dadurch gekennzeichnet, daß** der in den Verfahrensschritten a) und b) gebildete Ester ohne Isolierung und Aufreinigung direkt in die Lactonisierungsstufe c) eingesetzt wird.

16. Verfahren nach den Ansprüchen 9 bis 15, **dadurch gekennzeichnet, daß** die Verfahrensschritte a) bis c) kontinuierlich durchgeführt werden.

## Claims

1. A process for the preparation of L-ascorbic acid, which comprises lactonizing 2-keto-L-gulonic acid or a melt of C₃-C₆-alkyl 2-keto-L-gulonate with acid catalysis in the presence exclusively of a solvent or solvent mixture which is miscible with water and in situ forms a solvent in which the ascorbic acid formed has a solubility of less than 2 g/l at room temperature.

2. A process as claimed in claim 1, wherein the lactonization is carried out with catalysis by a mineral acid.

3. A process as claimed in either of claims 1 and 2, wherein the lactonization is catalyzed by hydrogen chloride or aqueous hydrochloric acid.

4. A process as claimed in any of claims 1 to 3, wherein the lactonization is carried out in the presence of a C₁-C₆-alcohol as solvent.

5. A process as claimed in either of claims 3 and 4, wherein C₁-C₆-alkyl halides are formed in situ from the alcohol.

6. A process as claimed in any of claims 3 to 5, wherein an alkyl halide of a C₁-C₆-alcohol is added to the reaction at the start or during the lactonization.

7. A process as claimed in any of claims 3 to 6, wherein an alkyl halide or a C₁-C₆-alcohol selected from the group consisting of ethyl chloride, propyl chloride, butyl chloride, tert-butyl chloride, pentyl chloride or hexyl chloride is added at the start or during the lactonization.

8. A process as claimed in any of claims 1 to 7, wherein the lactonization is carried out in a continuous reaction.

9. A process for the preparation of L-ascorbic acid, which comprises the following steps:
a) esterification of 2-keto-L-gulonic acid or 2,3:4,6-di-O-isopropylidene-2-keto-L-gulonic acid with a C₃-C₆-alcohol in the presence of an acidic catalyst,
b) distillation of the excess C₃-C₆-alcohol together with the water of reaction formed and
c) lactonization of the C₃-C₆-alkyl 2-keto-L-gulonate formed with acid catalysis in the presence exclusively of a solvent or solvent mixture which is miscible with water and in situ forms a solvent in which the ascorbic acid formed is poorly soluble.

10. A process as claimed in claim 9, wherein the esterification in process step a) is carried out using an alcohol selected from the group consisting of n-propanol, isopropanol, 1-butanol, 2-butanol, 2-methyl-1-propanol, 2-methyl-2-propanol, 1-pentanol or 1-hexanol.

11. A process as claimed in either of claims 9 and 10, wherein the esterification in process step a) is carried out in the presence of a mineral acid, of an acidic ion exchanger or of a catalyst attached to a support.

12. A process as claimed in any of claims 9 to 11, wherein the esterification in process step a) is carried out with n-butanol in the presence of sulfuric acid.

13. A process as claimed in any of claims 9 to 12, wherein the reaction temperatures in the process steps a) to c) are in the range from -10 to 160°C.

14. A process as claimed in any of claims 9 to 13, wherein the esterification and lactonization in process steps a) to c) are carried out at pressures in the range from 0.1 to 20 bar.

15. A process as claimed in any of claims 9 to 14, wherein the ester formed in process steps a) and b) is employed directly in lactonization step c) without isolation and purification.

16. A process as claimed in any of claims 9 to 15, wherein process steps a) to c) are carried out continuously.

## Revendications

1. Procédé pour la préparation de l'acide L-ascorbique, **caractérisé par le fait que** l'on lactonise l'acide 2-céto-L-gulonique ou une masse fondue de 2-céto-L-gulonate d'alkyle en C3-C6 avec catalyse par un acide en présence exclusivement d'un solvant ou mélange solvant qui est miscible à l'eau et forme in situ un milieu solvant dans lequel l'acide ascorbique formé a une solubilité inférieure à 2 g/l à température ambiante.

2. Procédé selon la revendication 1, **caractérisé par le fait que** l'on lactonise avec catalyse par un acide minéral.

3. Procédé selon l'une des revendications 1 ou 2, **caractérisé par le fait que** l'on catalyse la lactonisation par le chlorure d'hydrogène ou l'acide chlorhydrique aqueux.

4. Procédé selon les revendications 1 à 3, **caractérisé par le fait que** la lactonisation est réalisée en présence d'un solvant consistant en un alcool en C1-C6.

5. Procédé selon les revendications 3 ou 4, **caractérisé par le fait qu'**il se forme in situ, à partir de l'alcool, un halogénure d'alkyle en C1-C6.

6. Procédé selon les revendications 3 à 5, **caractérisé par le fait que**, au début de la lactonisation ou durant la lactonisation, on ajoute un halogénure d'alkyle dérivé d'un alcool en C1-C6.

7. Procédé selon les revendications 3 à 6, **caractérisé par le fait que**, au début de la lactonisation ou durant la lactonisation, on ajoute un halogénure d'alkyle dérivé d'un alcool en C1-C6 et choisi dans le groupe consistant en le chlorure d'éthyle, le chlorure de propyle, le chlorure de butyle, le chlorure de tert-butyle, le chlorure de pentyle ou le chlorure d'hexyle.

8. Procédé selon les revendications 1 à 7, **caractérisé par le fait que** la lactonisation est réalisée en continu.

9. Procédé pour la préparation de l'acide L-ascorbique **caractérisé par le fait qu'**il comprend les stades opératoires suivants :
a) estérification de l'acide 2-céto-L-gulonique ou de l'acide 2,3 :4,6-di-O-isopropylidène-2-céto-L-gulonique en présence d'un catalyseur acide, à l'aide d'un alcool en C3-C6,
b) distillation de l'excès d'alcool en C3-C6 et de l'eau formée dans la réaction et
c) lactonisation du 2-céto-L-gulonate d'alkyle en C3-C6 formé avec catalyse par un acide en présence exclusivement d'un solvant ou mélange solvant qui est miscible à l'eau et forme in situ un milieu solvant dans lequel l'acide ascorbique formé est peu soluble.

10. Procédé selon la revendication 9, **caractérisé par le fait que** l'estérification du stade opératoire a) est réalisée à l'aide d'un alcool choisi dans le groupe consistant en le n-propanol, l'isopropanol, le 1-butanol, le 2-butanol, le 2-méthyl-1-propanol, le 2-méthyl-2-propanol, le 1-pentanol ou le 1-hexanol.

11. Procédé selon la revendication 9 ou 10, **caractérisé par le fait que** l'estérification du stade opératoire a) est réalisée en présence d'un acide minéral, d'un échangeur d'ions acide ou d'un catalyseur fixé sur support:

12. Procédé selon les revendications 9 à 11, **caractérisé par le fait que** l'estérification du stade opératoire a) est réalisée à l'aide du n-butanol en présence d'acide sulfurique.

13. Procédé selon les revendications 9 à 12, **caractérisé par le fait que** les températures de réaction dans les stades opératoires a) à c) se situent dans l'intervalle de -10 à +160°C.

14. Procédé selon les revendications 9 à 13, **caractérisé par le fait que** l'estérification et la lactonisation des stades opératoires a) à c) sont réalisés à des pressions dans l'intervalle de 0,1 à 20 bar.

15. Procédé selon les revendications 9 à 14, **caractérisé par le fait que** l'on envoie l'ester formé dans les stades opératoires a) et b) directement, sans isolement ni purification, au stade de lactonisation c).

16. Procédé selon les revendications 9 à 15, **caractérisé en ce que** les stades opératoires a) à c) sont réalisés en continu.
